(19) Europäisches Patentamt / European Patent Office / Office européen des brevets

(11) **EP 4 129 272 B1**

(12) **EUROPEAN PATENT SPECIFICATION**

(45) Date of publication and mention
of the grant of the patent:
**11.09.2024 Bulletin 2024/37**

(21) Application number: **22460020.5**

(22) Date of filing: **04.04.2022**

(51) International Patent Classification (IPC):
**A61K 9/14** *(2006.01)*     **A61K 31/12** *(2006.01)*

(52) Cooperative Patent Classification (CPC):
**A61K 9/143;** A61K 36/9066

(54) **A METHOD OF APPLYING OF TURMERIC ON HIERARCHICAL ZEOLITES WITH A METHOD OF ITS PHOTODYNAMIC RELEASE**

VERFAHREN ZUM AUFBRINGEN VON KURKUMA AUF HIERARCHISCHEN ZEOLITHEN MIT EINEM VERFAHREN ZU SEINER FOTODYNAMISCHEN FREISETZUNG

PROCÉDÉ D'APPLICATION DE CURCUMA SUR DES ZÉOLITES HIÉRARCHIQUES AVEC SON PROCÉDÉ DE LIBÉRATION PHOTODYNAMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.08.2021 PL 43867021**
**04.08.2021 PL 43867321**

(43) Date of publication of application:
**08.02.2023 Bulletin 2023/06**

(73) Proprietor: **Uniwersytet Im. Adama Mickiewicza W Poznaniu**
**61-712 Poznan (PL)**

(72) Inventors:
• **Musielak, Ewelina**
  **63-800 Gostyn (PL)**
• **Feliczak-Guzik, Agnieszka**
  **62-272 Lagiewniki Koscielne (PL)**
• **Nowak, Izabela**
  **62-020 Swarzedz (PL)**

(74) Representative: **Urbanska-Luczak, Barbara**
**Kancelaria Patentowa TAX - PAT**
**B. Urbanska-Luczak, J.Luczak**
**ul. Kosciuszki 103/1**
**61-717 Poznan (PL)**

(56) References cited:
**WO-A1-2015/191962     US-A1- 2010 291 387**
**US-A1- 2020 155 480**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The subject of the invention is a method of applying turmeric on hierarchical zeolites possessing secondary porosity, pores in the range of 2-50 nm, synthesised on the basis of faujasite and with the use of ionic or the non-ionic structure-forming agents, with a method for photo-dynamic release of turmeric from the hierarchical zeolite.

U.S. Patent application 2020/155480 A1, discloses a methods for drug delivery, tumor imaging, and oxidative dehydrogenation using hierarchical ZSM-5 complex. The invention is directed to hierarchical aluminosilicates that contain both micro- and meso-pores to methods for loading and delivering poorly soluble antioxidants such as CoQ10 and curcumin to subjects, and to a top-down method for producing hierarchical aluminosilicates.

A composition comprising curcumin and a hierarchical aluminosilicate having an $SiO_2/Al_2O_3$ ratio ranging from 22 to 80, and an external surface area of 295 to 785 m$^2$/g, a mesopore volume of from 0.11 to 0.78 cc/g, and a bimodal mesopore diameter distribution with a first pore diameter at 2.3 nm and a second pore diameter at 3.8 nm. The curcumin is adsorbed onto the hierarchical aluminosilicate in an amorphous form, wherein the hierarchical aluminosilicate has a curcumin adsorption of about 98%, and composition comprises at least 30 wt. % of the curcumin based on the total weight of the composition.

Hierarchical zeolites are materials which are characterised by possessing, in addition to micropores with a size of <2 nm according to IUPAC classification (primary porosity), secondary porosity, which results in the presence of at least one, additional system of pores, mainly in the mesopores range (pores size from 2 nm to 50 nm according to IUPAC classification). These materials demonstrate a range of uses, e.g. in catalysis [A. Feliczak-Guzik, Hierarchical zeolites: Synthesis and catalytic properties, Micropor. Mesopor. Mat., 2018, 259, 33-45], adsorption [H. Qu, Y. Ma, B. Li, L. Wang, Hierarchical zeolites: synthesis, structural control, and catalytic applications, Emerg. Mat. 2020, 3, 225-245] or in medicine [A. G. Abd-Elsatar, M. M. Farag, H. F. Youssef, S. A. Salih, M. M. Mounier, E. El-Meliegy, Different zeolite systems for colon cancer therapy: monitoring of ion release, cytotoxicity and drug release behavior, Progress in Biomat (2019) 8:101-113]. These materials may be used as carriers for active substances, enabling therefore their prolonged release and transport to specific tissues and organs [Therapeutic and prophylactic compositions including catalytic biomimetic solids and methods to prepare and use them, WO2001012221A1].

The requirement for prolonged release carriers of active substances is for them to be effective and safe. Moreover in such carriers the dependency between dose and therapeutic effect of the active substance must be well established. Additionally, the effect obtained by its administration should be at least comparable with the effect obtained by using a conventional form of the medicine/supplement, while simultaneously reducing the adverse effects [M. J. Rathbone, J. Hadgraft, M. S. Roberts, M. E. Lane: Modified-release drug delivery technology - edition II. Vol 1 - Informa. New York 2008. 4. M. Sznitowska, W. Zebrowska: Doustne postacie leku o modyfikowanym uwalnianiu. Cz. I - Tabletki o przedfuionym uwalnianiu - (Oral forms of modified release medication, part I - prolonged release tablets) Ordynator Leków. 2003, 3(5), 3-14]. Therefore, an important element of therapy is delivering the active substance to a specific location in the organism and releasing it for a desired duration. For this purpose, the necessary drug carriers are used, which may be carbon, polymer or porous materials [A. Ramila, B. Munoz, J. Pérez-Pariente, M. Vallet-Regí, J. Sol-Gel Sci. Technol., 2003, 26, 1199-1202; P. Horcajada, A. Rámila, J. Pérez-Pariente, M. Vallet-Regi, Micropor. Mesopor. Mat., 2004, 68, 105-109].

The main advantage of using zeolites as matrices for the application of medicinal substances, with the possibility of their release in a permanent manner result from their unique properties [A. G. Abd-Elsatar, M. M. Farag, H. F. Youssef, S. A. Salih, M. M. Mounier, E. El-Meliegy, Different zeolite systems for colon cancer therapy: monitoring of ion release, cytotoxicity and drug release behavior, Progress in. 2019, 8:101-113]. These materials are characterised by high biocompatibility and non-toxicity for various types of cells and tissues [M. Kralj, K. Pavelic, Medicine on a small scale: how molecular medicine can benefit from self-assembled and nanostructured materials. EMBO Rep 2003, 4, 1008-1012. D. C. Thom, J. E. Davies, J. P. Santerre, S. Friedman, The haemolytic and cytotoxic properties of a zeolite-containing root filling material in vitro. Oral Surg Oral Med Oral Pathol Oral Radiol Endod 2003, 95, 101-108] and are successfully used as the carriers of various therapeutic substances [E. Khodaverdi, H. A. Soleimani, F. Mohammadpour, F. Hadizadeh, Synthetic zeolites as controlled-release delivery systems for anti-inflammatory drugs. Chem Biol Drug Des., 2016, 87, 849-857].

Turmeric is a substance isolated from the root of Indian saffron *Curcuma longa,* a natural substance in the group of polyphenols. Due to its ability to regulate many important transcription factors, cytokines, protein kinases and adhesive properties of particles, turmeric has found use as anti-inflammatory, anti-oxidant, anti-proliferative, anti-angiogenic and anti-cancer agent. Due to these properties it is currently used in the treatment of various diseases, such as cardiovascular diseases, diabetes, asthma and bronchitis, neurodegenerative diseases, rheumatoid arthritis, psoriasis, AIDS, but is mainly used in the treatment of cancers [A. Goel, A. B. Kunnumakkara, B. B. Aggarwal, Biochem Pharmacol. 75 (2008) 787-809; K. Nagahama, T. Utsumi, T. Kumano, S. Maekawa, N. Oyama, J. Kawakami, Sci Rep. 60, (2016) 30962; Y. S. Kim, Y. Ahn, S. H. Hong, S. Y. Joo, K. H. Kim, I. S. Sohn, H. W. Park, Y. J. Hong, J. H. Kim, W. Kim, M. H. Jeong, J. G. Cho, J. C. Park, J. C. Kang, Jour. Cardiovasc. Pharmacol. 50, (2007) 41; X. L. Jun, H. Xiang-Fe, L. Z.

Hang, Anti-Cancer Agents in Medical Chem. 12 (2012) 210].

Synergistic activity of zeolites and curcumin extract was described in [Food supplements, drug or medicine product contains a portion of zeolite for tissue detoxification and/pollutant excretion, where the food supplement contains a portion of curcumin extract, DE102011114455A1]. Zeolite (clinoptilolite) due to its porosity demonstrates the ability of ion exchange and of binding toxins. Additionally, the use of zeolite slows down ageing processes, protects against free radicals and helps in reduce inflammation.

Metal-organic frameworks (MOF) type ZIF-8 (topologically isomorphic with zeolites) encapsulated with turmeric have demonstrated activity in cervical cancer [M. Zheng, S. Liu, X. Guan, Z. Xie, ACS Applied Materials & Interfaces 7(40), 2015].

In the group of Shoba et al. it was indicated that piperine, which is a hepatic and intestinal glucuronidation inhibitor, impacts the bioavailability of turmeric in healthy persons. After administration of 2 g of turmeric with 20 mg of piperine a positive effect was observed, that is piperine has increased the bioavailability of turmeric by as many as 2000%. Based on obtained studies it was demonstrated that piperine increases the serum concentration, absorption and bioavailability of turmeric in humans, without adverse effects [Shoba G., Joy D., Joseph T., Majeed M., Rajendran R., Srinivas PS.; Planta Medica 64 (1998) 353-356].

Moreover, turmeric is also a potential active substance used in photodynamic therapies. The American National Institutes of Health (NIH) agency defines photodynamic therapies as one of the methods used in the treatment of cancer, through the use of active substances, called "photosensitizers" and of radiation with precisely determined energy. This method is an alternative to traditional cancer treatment methods, that is, radio-, chemo- and hormone therapy. It may be also used as complementary therapy [http://www.cancer.gov/cancertopics/factsheet/Therapy/photodynamic - downloaded on 14.07.2021]. Therefore, the idea of the invention is to create a photodynamic method of prolonged release of turmeric with anti-cancer properties from hierarchical zeolites.

The goal of the invention is to create a method of applying turmeric on hierarchical zeolites characterised by secondary porosity with size in the range of 2-50 nm, which were synthesised on the basis of faujasite and ionic (e.g. hexadecyltrimethylammonium bromide) and non-ionic structure-forming agents (e.g. polyethylene glycol octadecyl ether or Pluronic F127), which enables obtaining hierarchical zeolite with an additional system of pores with applied turmeric for photodynamic, delayed release from the hierarchic zeolite caused by visible light. This invention concerns pharmaceutical pre-formulation (medicinal product) appropriate for the creation of solid galenic forms, e.g. for tabletting.

The essence of the invention is a method of applying turmeric on hierarchical zeolites possessing secondary porosity, pores in the range 2-50 nm, synthesised on the basis of faujasite and either non-ionic structure forming agents selected from the group of polyethylene glycol octadecyl ether or Pluronic F127 or the ionic structure-forming agent hexadecyltrimethylammonium bromide in the presence or absence of piperine, the following is added:

- a solvent, advantageously acetone in an amount of 2.5-12.5% w/w, advantageously 12.5% w/w in relation to hierarchical zeolite,
- 20-60% w/w of turmeric, advantageously 20% w/w of turmeric in relation to hierarchical zeolite, and
- 0.1-0.4% w/w of piperine, advantageously 0.1% w/w of piperine in relation to hierarchical zeolite,
  whereas the entire process of mixing is conducted for 24-30 h advantageously 24 h in a temperature of 20-40°C advantageously in 23°C, and
- washing the obtained product with a water-ethanol solution in a volumetric relation of 1:1 and
- drying afterwards.

The material obtained in this manner is subjected to photodynamic release of turmeric.

A method according to the invention is a method of photodynamic release of turmeric from hierarchical zeolite, consisting of:

- adding phosphate buffer with a pH ranging from 5 to 6, advantageously 5.8 to hierarchical zeolite obtained from faujasite type zeolite with applied turmeric turmeric, and characterised by secondary porosity, with pores in the range of 2 to 50 nm, obtained on the basis of faujasite type zeolite and ionic structure-forming agents e.g. hexadecyltrimethylammonium bromide, with turmeric in an amount of 20% to 60% w/w, advantageously 60% w/w in relation to hierarchical zeolite, with the amount of buffer being 1:5, 1:10 and 1:15 by weight compared to hierarchical zeolite with applied turmeric, advantageously 1:10 by weight, and

- adding a penetration promoter in an amount of 5% to 25% w/w in relation to the buffer, where the process of releasing of turmeric from hierarchical zeolite is conducted for 0.5 to 30 h in a temperature of 35 to 43°C, advantageously 37°C with the use of UV-Vis in the wavelength range of 400 to 800 nm, in order to obtain the product.

It is advantageous when the penetration promoter is glycerine or ethyl alcohol or Tween 80.

The method according to the invention demonstrates a number of advantages, in particular, turmeric may be processed up to a high degree of loading. The method according to the invention uses materials synthesised in a simpler manner than the MOF material. An undesirable effect of caking of the carrier is not observed, and thus using the method according to the invention enables simple manufacturing of tablets for therapeutic (pharmaceutical) purposes. The method according to the invention ensures a higher degree of release of active substance in conditions of expected working temperatures, and the release process is caused by visible light. Due to the low temperature at which the process is conducted, the impact on temperature on the activity of the active substance is very low in case of the method according to the invention.

Unexpectedly, it turned out that turmeric may be in a simple manner applied to a solid carrier with a uniform distribution of pores with the use of created method of application. It turned out that the hierarchical zeolite on the basis of faujasite and either non-ionic structure-forming agents or ionic structure-forming agents may be obtained using a known practical chemistry of material [A. Feliczak-Guzik, M. Sprynskyy, I. Nowak, M. Jaroniec, B. Buszewski, Journal of Colloid and Interface Science 2018, 516, 379-383; A. Feliczak-Guzik, M. Sprynskyy, I. Nowak, B. Buszewski, Catalysts 2018, 8, 31-43, patent application: A. Feliczak-Guzik, I. Nowak, E. Musielak P.433556 [WIPO ST 10/C PL433556]. According to the invention to a mixture containing hierarchical zeolite characterised by secondary porosity with pore size in the range of 2-50 nm, obtained on the basis of commercial faujasite type zeolite and ionic structure-forming agent e.g. hexadecyltrimethylammonium bromide or non-ionic structure forming agent e.g. polyethylene glycol octadecyl ether or Pluronic F127 a solvent is added in an amount of 2.5-12.5% w/w in relation to hierarchical zeolite, 20-60% w/w of turmeric in relation to hierarchical zeolite and 0.1-0.4% w/w of piperine in relation to hierarchical zeolite at a temperature of 20-40°C. The entire mixture is transferred to a magnetic stirrer and stirred for 24-30 h in a temperature of 20-40°C, and then the product is separated from the mixture in a known manner. This pharmaceutical pre-formulation may be conveniently galenically processed, in particular by tabletting them into solid pharmaceutical preparations (medicinal products). In the method according to the invention ethyl alcohol, acetone, n-butanol and a mixture of acetone with the aforementioned solvents were used as a solvent.

Additionally it turned out that the hierarchical zeolite with the applied active substance (turmeric) may be subjected to photodynamic release of the active substance. According to the invention, a phosphate buffer is added to a mixture containing hierarchical zeolite characterised by secondary porosity with pore sizes in the range of 2 to 50 nm, obtained on the basis of commercial faujasite type zeolite and ionic structure-forming agent, e.g. hexadecyltrimethylammonium bromide along with applied turmeric (from 20 to 60% w/w in relation to hierarchical zeolite), with the amount of buffer being 1:5; 1:10 and 1:15 in weight by weight relation to hierarchical zeolite with applied turmeric, advantageously 1:10 with a pH ranging from 5 to 6, from 5 to 25% w/w in relation to glycerine at a temperature from 35 to 43°C. The entire mixture is transferred to a magnetic stirrer and stirred for 0.5-30 h in a temperature of 35-43°C with the use of UV-VIS radiation and without access of light.

The product obtained using the method according to the invention is characterised by a high turmeric load, which was presented in Table 1 and a high degree of release of turmeric, which was presented in Table 2.

The invention is illustrated by the examples below:

**Example 1**

To hierarchical zeolite characterised by secondary porosity with the pore sizes in the range of 2-50 nm, obtained on the basis of ionic structure-forming agent (hexadecyltrimethylammonium bromide) a 20% w/w of turmeric was added and 12.5% w/w of acetone was added in relation to hierarchical zeolite. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

**Example 2**

Procedure was the same as in example 1, changing the stirring temperature to 20°C.

**Example 3**

Procedure was the same as in example 1, changing the stirring temperature to 40°C.

**Example 4**

Procedure was the same as in example 1, changing the stirring time to 30 h.

**Example 5**

Procedure was the same as in example 1, changing the amount of acetone to 2.5% w/w in relation to hierarchical zeolite.

**Example 6**

Procedure was the same as in example 1, changing the type of solvent to ethyl alcohol, 2.5% w/w in relation to hierarchical zeolite.

**Example 7**

Procedure was the same as in example 1, changing the type of solvent to ethyl alcohol, 12.5% w/w in relation to hierarchical zeolite.

**Example 8**

Procedure was the same as in example 1, changing type of solvent to n-butanol 2.5% w/w in relation to hierarchical zeolite.

**Example 9**

Procedure was the same as in example 1, changing the type of solvent to n-butanol 12.5% w/w in relation to hierarchical zeolite.

**Example 10**

Procedure was the same as in example 1, changing the amount of turmeric to 40% w/w in relation to hierarchical zeolite.

**Example 11**

Procedure was the same as in example 1, changing the amount of turmeric to 60% w/w in relation to hierarchical zeolite.

**Example 12**

To hierarchical zeolite characterised by secondary porosity with the pore sizes in the range of 2-50 nm, obtained on the basis of non-ionic structure-forming agent (polyethylene glycol octadecyl ether) a 20% w/w in relation to hierarchical zeolite of turmeric was added and 12.5% w/w in relation to hierarchical zeolite of acetone was added. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

**Example 13**

Procedure was the same as in example 12, changing the amount of turmeric to 40% w/w in relation to hierarchical zeolite.

**Example 14**

Procedure was the same as in example 12, changing the amount of turmeric to 60% w/w in relation to hierarchical zeolite.

**Example 15**

Procedure was the same as in example 12, changing the amount of acetone to 2.5% w/w in relation to hierarchical zeolite.

**Example 16**

Procedure was the same as in example 12, changing the type of solvent to ethyl alcohol, 2.5% w/w in relation to hierarchical zeolite.

**Example 17**

Procedure was the same as in example 12, changing the type of solvent to ethyl alcohol, 12.5% w/w in relation to hierarchical zeolite.

**Example 18**

Procedure was the same as in example 12, changing the type of solvent to n-butanol 2.5% w/w in relation to hierarchical zeolite.

**Example 19**

Procedure was the same as in example 12, changing the type of solvent to n-butanol 12.5% w/w in relation to hierarchical zeolite.

**Example 20**

Procedure was the same as in example 12, changing the stirring temperature to 20°C.

**Example 21**

Procedure was the same as in example 12, changing the stirring temperature to 40°C.

**Example 22**

Procedure was the same as in example 12, changing the stirring time to 30 h.

**Example 23**

To hierarchical zeolite characterised by secondary porosity with the pore sizes in the range of 2-50 nm, obtained on the basis of non-ionic structure-forming agent (Pluronic F127) a 20% w/w in relation to hierarchical zeolite of turmeric was added and 12.5% w/w in relation to hierarchical zeolite of acetone was added. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

### Example 24

Procedure was the same as in example 23, changing the amount of turmeric to 40% w/w in relation to hierarchical zeolite.

### Example 25

Procedure was the same as in example 23, changing the amount of turmeric to 60% w/w in relation to hierarchical zeolite.

### Example 26

Procedure was the same as in example 23, changing the amount of acetone to 2.5% w/w in relation to hierarchical zeolite.

### Example 27

Procedure was the same as in example 23, changing the type of solvent to ethyl alcohol, 2.5% w/w in relation to hierarchical zeolite.

### Example 28

Procedure was the same as in example 23, changing the type of solvent to ethyl alcohol, 12.5% w/w in relation to hierarchical zeolite.

### Example 29

Procedure was the same as in example 23, changing the type of solvent to n-butanol 2.5% w/w in relation to hierarchical zeolite.

### Example 30

Procedure was the same as in example 23, changing the type of solvent to n-butanol 12.5% w/w in relation to hierarchical zeolite.

### Example 31

Procedure was the same as in example 23, changing the stirring temperature to 20°C.

### Example 32

Procedure was the same as in example 23, changing the stirring temperature to 40°C.

### Example 33

Procedure was the same as in example 23, changing the stirring time to 30 h.

### Example 34

To hierarchical zeolite characterised by secondary porosity with the pore sizes in the range of 2-50 nm, obtained on the basis of ionic structure-forming agent (hexadecyltrimethylammonium bromide) a 20% w/w in relation to hierarchical zeolite of turmeric and 0.1% w/w in relation to hierarchical zeolite of piperine was added and 12.5% w/w in relation to hierarchical zeolite of acetone was added. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

**Example 35**

Procedure was the same as in example 34, changing the amount of piperine to 0.2% w/w in relation to hierarchical zeolite.

**Example 36**

Procedure was the same as in example 34, changing the amount of piperine to 0.4% w/w in relation to hierarchical zeolite.

**Example 37**

To hierarchical zeolite characterised by secondary porosity with the pore sizes in the range of 2-50 nm, obtained on the basis of non-ionic structure-forming agent (polyethylene glycol octadecyl ether) a 20% w/w in relation to hierarchical zeolite of turmeric and 0.1% w/w in relation to hierarchical zeolite of piperine was added and 12.5% w/w in relation to hierarchical zeolite of acetone was added. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

**Example 38**

Procedure was the same as in example 37, changing the amount of piperine to 0.2% w/w in relation to hierarchical zeolite.

**Example 39**

Procedure was the same as in example 37, changing the amount of piperine to 0.4% w/w in relation to hierarchical zeolite.

**Example 40**

To hierarchical zeolite characterised by secondary porosity with the pore sizes in the range of 2-50 nm, obtained on the basis of non-ionic structure-forming agent (Pluronic F127) a 20% w/w in relation to hierarchical zeolite of turmeric and 0.1% w/w in relation to hierarchical zeolite of piperine was added and 12.5% w/w in relation to hierarchical zeolite of acetone was added. The entire mixture was stirred for 24 h in a temperature of 23°C in a magnetic stirrer. The obtained product was filtered and washed three times with a water-ethanol mixture in a 1:1 relationship by volume and air dried for 24 h. The degree of turmeric loading was calculated using the formula:

$$\text{Turmeric loading degree [\%]} = 100 \times \frac{\text{mass of turmeric loaded into the carrier [mg]}}{\text{mass of hierarchical zeolite together with turmeric [mg]}}$$

**Example 41**

Procedure was the same as in example 40, changing the amount of piperine to 0.2% w/w in relation to hierarchical zeolite.

**Example 42**

Procedure was the same as in example 40, changing the amount of piperine to 0.4% w/w in relation to hierarchical zeolite.

**Example 43**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 60% w/w in relation to hierarchical zeolite a phosphate buffer with a pH of 5.8 was added in an amount of 1:10 by weight in relation to hierarchical zeolite with the applied turmeric. The turmeric release process was conducted for 24 h at a temperature of 37°C on a magnetic stirrer with the use of visible light (radiation wavelength range of 400-800 nm).

The degree of turmeric release was established with the use of UV-Vis spectrophotometer, by measuring the adsorption of turmeric in the buffer mixture. It was calculated on the basis of the following formula:

$$\% \text{ release of turmeric} = \frac{A_p}{A_w}\left(\frac{m_w[mg] \; x \; C_w}{V_w \; [ml]}\right)\left(\frac{1}{D_w}\right)\left(\frac{V_p \; [ml]}{m_p \; [mg]}\right) x \; 100\%$$

$A_p$ - absorbance of the sample
$A_w$ - absorbance of the reference
$m_w$ - mass of the reference
$m_p$ - mass of the active substance in the sample
$C_w$ - purity of the reference
$D_w$ - dilution of the reference
$V_w$ - volume of the reference solution
$V_p$ - volume of the phosphate buffer

**Example 44**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 60% w/w in relation to hierarchical zeolite a phosphate buffer with a pH of 5.8 was added in an amount of 1:10 by weight in relation to hierarchical zeolite with the applied turmeric and 25% w/w in relation to phosphate buffer of penetration promoter - glycerine was added. The turmeric release process was conducted for 24 h at a temperature of 37°C on a magnetic stirrer with the use of visible light (radiation wavelength range of 400-800 nm).

The degree of turmeric release was established with the use of UV-Vis spectrophotometer, by measuring the adsorption of turmeric in the buffer mixture with the penetration promoter. It was calculated on the basis of the following formula:

$$\% \text{ release of turmeric} = \frac{A_p}{A_w}\left(\frac{m_w[mg] \; x \; C_w}{V_w \; [ml]}\right)\left(\frac{1}{D_w}\right)\left(\frac{V_p \; [ml]}{m_p \; [mg]}\right) x \; 100\%$$

$A_p$ - absorbance of the sample
$A_w$ - absorbance of the reference
$m_w$ - mass of the reference
$m_p$ - mass of the active substance in the sample
$C_w$ - purity of the reference
$D_w$ - dilution of the reference
$V_w$ - volume of the reference solution
$V_p$ - volume of the phosphate buffer

**Example 45**

Procedure was the same as in example 44, adding phosphate buffer with a pH of 5.0 in an amount of 1:10 by weight in relation to hierarchical zeolite with the applied turmeric.

**Example 46**

Procedure was the same as in example 44, adding phosphate buffer with a pH of 6.0 in an amount of 1:10 by weight in relation to hierarchical zeolite with the applied turmeric.

**Example 47**

Procedure was the same as in example 44, adding phosphate buffer with a pH of 5.8 in an amount of 1:5 by weight in relation to hierarchical zeolite with the applied turmeric.

**Example 48**

Procedure was the same as in example 44, adding phosphate buffer with a pH of 5.8 in an amount of 1:15 by weight in relation to hierarchical zeolite with the applied turmeric.

**Example 49**

Procedure was the same as in example 44, adding glycerine 5% w/w in relation to phosphate buffer.

**Example 50**

Procedure was the same as in example 44, changing the type of penetration promoter to ethyl alcohol, 25% w/w in relation to phosphate buffer.

**Example 51**

Procedure was the same as in example 50, adding ethyl alcohol 5% w/w in relation to phosphate buffer.

**Example 52**

Procedure was the same as in example 44, changing the type of penetration promoter to Tween 80, 25% w/w in relation to phosphate buffer.

**Example 53**

Procedure was the same as in example 52, adding Tween 80 5% w/w in relation to phosphate buffer.

**Example 54**

Procedure was the same as in example 44, changing the turmeric release process time to 0.5 h.

**Example 55**

Procedure was the same as in example 44, changing the turmeric release process time to 30 h.

**Example 56**

Procedure was the same as in example 44, changing the turmeric release process temperature to 35°C.

**Example 57**

Procedure was the same as in example 44, changing the turmeric release process temperature to 43°C.

**Example 58**

Procedure was the same as in example 44, changing the wavelength range of radiation to 200 to 400 nm.

**Example 59**

Procedure was the same as in example 44, conducting turmeric release process without access of light.

**Example 60**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 40% w/w in relation to hierarchical zeolite a phosphate buffer with a pH of 5.8 was added in an amount of 1:10 by weight in relation to hierarchical zeolite with the applied turmeric. The turmeric release process was conducted for 24 h at a temperature of 37°C on a magnetic stirrer with the use of visible light (radiation wavelength range of 400-800 nm). The degree of turmeric release was established with the use of UV-Vis spectrophotometer, by measuring the adsorption of turmeric in the buffer mixture.

**Example 61**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 40% w/w in relation to hierarchical zeolite a phosphate buffer with a pH of 5.8 was added in an amount of 1:10 by weight in relation to hierarchical zeolite with the applied turmeric and 25% w/w in relation to phosphate buffer of penetration promoter - glycerine was added. The turmeric release process was conducted for 24 h at a temperature of 37°C on a magnetic stirrer with the use of visible light (radiation wavelength range of 400-800 nm). The degree of turmeric release was established with the use of UV-Vis spectrophotometer, by measuring the adsorption of turmeric in the buffer mixture with the penetration promoter.

**Example 62**

Procedure was the same as in example 61, adding phosphate buffer with a pH of 5.0 in an amount of 1:10 by weight in relation to hierarchical zeolite with the applied turmeric.

**Example 63**

Procedure was the same as in example 61, adding phosphate buffer with a pH of 6.0 in an amount of 1:10 by weight in relation to hierarchical zeolite with the applied turmeric.

**Example 64**

Procedure was the same as in example 61, adding phosphate buffer with a pH of 5.8 in an amount of 1:5 by weight in relation to hierarchical zeolite with the applied turmeric.

**Example 65**

Procedure was the same as in example 61, adding phosphate buffer with a pH of 5.8 in an amount of 1:15 by weight in relation to hierarchical zeolite with the applied turmeric.

**Example 66**

Procedure was the same as in example 61, adding glycerine 5% w/w in relation to phosphate buffer.

**Example 67**

Procedure was the same as in example 61, changing the type of penetration promoter to ethyl alcohol, 25% w/w in relation to phosphate buffer.

**Example 68**

Procedure was the same as in example 61, adding ethyl alcohol 5% w/w in relation to phosphate buffer.

**Example 69**

Procedure was the same as in example 61, changing the type of penetration promoter to Tween 80, 25% w/w in relation to phosphate buffer.

**Example 70**

Procedure was the same as in example 69, adding Tween 80 5% w/w in relation to phosphate buffer.

**Example 71**

Procedure was the same as in example 61, changing the turmeric release process time to 0.5 h.

**Example 72**

Procedure was the same as in example 61, changing the turmeric release process time to 30 h.

**Example 73**

Procedure was the same as in example 61, changing the turmeric release process temperature to 35°C.

**Example 74**

Procedure was the same as in example 61, changing the turmeric release process temperature to 43°C.

**Example 75**

Procedure was the same as in example 61, changing the wavelength range of radiation to 200 to 400 nm.

**Example 76**

Procedure was the same as in example 61, conducting the turmeric release process without access of light.

**Example 77**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 20% w/w in relation to hierarchical zeolite a phosphate buffer with a pH of 5.8 was added in an amount of 1:10 by weight in relation to hierarchical zeolite with the applied turmeric. The turmeric release process was conducted for 24 h at a temperature of 37°C on a magnetic stirrer with the use of visible light (radiation wavelength range of 400-800 nm). The degree of turmeric release was established with the use of UV-Vis spectrophotometer, by measuring the adsorption of turmeric in the buffer mixture.

**Example 78**

To hierarchical zeolite characterised by secondary porosity with the pore sizes in the range of 2-50 nm, obtained on the basis of ionic structure-forming agent (e.g. hexadecyltrimethylammonium bromide) with applied turmeric in an amount of 20% w/w in relation to hierarchical zeolite a phosphate buffer with a pH of 5.8 was added in an amount of 1:10 by weight in relation to hierarchical zeolite with the applied turmeric and 25% w/w in relation to phosphate buffer of penetration promoter - glycerine was added. The turmeric release process was conducted for 24 h at a temperature of 37°C on a magnetic stirrer with the use of visible light (radiation wavelength range of 400-800 nm). The degree of turmeric release was established with the use of UV-Vis spectrophotometer, by measuring the adsorption of turmeric in the buffer mixture with the penetration promoter.

**Example 79**

Procedure was the same as in example 78, adding phosphate buffer with a pH of 5.0 in an amount of 1:10 by weight in relation to hierarchical zeolite with the applied turmeric.

**Example 80**

Procedure was the same as in example 78, adding phosphate buffer with a pH of 6.0 in an amount of 1:10 by weight in relation to hierarchical zeolite with the applied turmeric.

**Example 81**

Procedure was the same as in example 78, adding phosphate buffer with a pH of 5.8 in an amount of 1:5 by weight in relation to hierarchical zeolite with the applied turmeric.

**Example 82**

Procedure was the same as in example 78, adding phosphate buffer with a pH of 5.8 in an amount of 1:15 by weight in relation to hierarchical zeolite with the applied turmeric.

**Example 83**

Procedure was the same as in example 78, adding glycerine 5% w/w in relation to phosphate buffer.

**Example 84**

Procedure was the same as in example 78, changing the type of penetration promoter to ethyl alcohol, 25% w/w in relation to phosphate buffer.

**Example 85**

Procedure was the same as in example 84, adding ethyl alcohol 5% w/w in relation to phosphate buffer.

**Example 86**

Procedure was the same as in example 78, changing the type of penetration promoter to Tween 80, 25% w/w in relation to phosphate buffer.

**Example 87**

Procedure was the same as in example 86, adding Tween 80 5% w/w in relation to phosphate buffer.

**Example 88**

Procedure was the same as in example 78, changing the turmeric release process time to 0.5 h.

**Example 89**

Procedure was the same as in example 78, changing the turmeric release process time to 30 h.

**Example 90**

Procedure was the same as in example 78, changing the turmeric release process temperature to 35°C.

**Example 91**

Procedure was the same as in example 78, changing the turmeric release process temperature to 43°C.

**Example 92**

Procedure was the same as in example 78, changing the wavelength range of radiation to 200 to 400 nm.

**Example 93**

Procedure was the same as in example 78, conducting the turmeric release process without access of light.

According to the examples 1-42 a high degree of turmeric loading was obtained, as demonstrated in table 1

Table 1 Degree of turmeric loading on hierarchical zeolites

| Example | Material | Amount of turmerc [% w/w] | Amount of piperine [% w/w] | Stirring time [h] | Stirring temperature [°C] | Type/ amount of solvent [% w/w] | Turmeric loading degree [%] |
|---|---|---|---|---|---|---|---|
| 1 | Hierarchical zeolite_ hexadecyltrimethylammonium bromide | 20.00 | - | 24.00 | 23.00 | Acetone / 12.50 | 39.20 |
| 2 | Hierarchical zeolite_ hexadecyltrimethylammonium bromide | 20.00 | - | 24.00 | 20.00 | Acetone / 12.50 | 36.40 |
| 3 | Hierarchical zeolite_ hexadecyltrimethylammonium bromide | 20.00 | - | 24.00 | 40.00 | Acetone / 12.50 | 30.06 |
| 4 | Hierarchical zeolite_ hexadecyltrimethylammonium bromide | 20.00 | - | 30.00 | 23.00 | Acetone / 12.50 | 39.80 |
| 5 | Hierarchical zeolite_ hexadecyltrimethylammonium bromide | 20.00 | - | 24.00 | 23.00 | Acetone / 2.50 | 30.01 |
| 6 | Hierarchical zeolite_ hexadecyltrimethylammonium bromide | 20.00 | - | 24.00 | 23.00 | Ethyl alcohol/ 2.50 | 24.90 |
| 7 | Hierarchical zeolite_ hexadecyltrimethylammonium bromide | 20.00 | - | 24.00 | 23.00 | Ethyl alcohol/ 12.50 | 28.50 |
| 8 | Hierarchical zeolite_ hexadecyltrimethylammonium bromide | 20.00 | - | 24.00 | 23.00 | n-butanol/ 2.50 | 18.05 |
| 9 | Hierarchical zeolite_ hexadecyltrimethylammonium bromide | 20.00 | - | 24.00 | 23.00 | n-butanol/ 12.50 | 22.04 |
| 10 | Hierarchical zeolite_ hexadecyltrimethylammonium bromide | 40.00 | - | 24.00 | 23.00 | Acetone / 12.50 | 61.80 |
| 11 | Hierarchical zeolite_ hexadecyltrimethylammonium bromide | 60.00 | - | 24.00 | 23.00 | Acetone / 12.5 | 87.60 |
| 12 | Hierarchical zeolite_ polyethylene glycol octadecyl ether | 20.00 | - | 24.00 | 23.00 | Acetone / 12.50 | 41.50 |
| 13 | Hierarchical zeolite_ polyethylene glycol octadecyl ether | 40.00 | - | 24.00 | 23.00 | Acetone / 12.50 | 68.10 |

(continued)

| Example | Material | Amount of turmerc [% w/w] | Amount of piperine [% w/w] | Stirring time [h] | Stirring temperature [°C] | Type/ amount of solvent [% w/w] | Turmeric loading degree [%] |
|---------|----------|---------------------------|----------------------------|-------------------|---------------------------|----------------------------------|------------------------------|
| 14 | Hierarchical zeolite_ polyethylene glycol octadecyl ether | 60.00 | - | 24.00 | 23.00 | Acetone / 12.50 | 89.60 |
| 15 | Hierarchical zeolite_ polyethylene glycol octadecyl ether | 20.00 | - | 24.00 | 23.00 | Acetone / 2.50 | 28.00 |
| 16 | Hierarchical zeolite_ polyethylene glycol octadecyl ether | 20.00 | - | 24.00 | 23.00 | Ethyl alcohol/ 2.50 | 23.00 |
| 17 | Hierarchical zeolite_ polyethylene glycol octadecyl ether | 20.00 | - | 24.00 | 23.00 | Ethyl alcohol/ 12.5 0 | 26.50 |
| 18 | Hierarchical zeolite_ polyethylene glycol octadecyl ether | 20.00 | - | 24.00 | 23.00 | n-butanol/ 2.50 | 17.00 |
| 19 | Hierarchical zeolite_ polyethylene glycol octadecyl ether | 20.00 | - | 24.00 | 23.00 | n-butanol/ 12.5 | 19.45 |
| 20 | Hierarchical zeolite_ polyethylene glycol octadecyl ether | 20.00 | - | 24.00 | 20.00 | Acetone / 12.50 | 35.03 |
| 21 | Hierarchical zeolite_ polyethylene glycol octadecyl ether | 20.00 | - | 24.00 | 40.00 | Acetone / 12.50 | 29.40 |
| 22 | Hierarchical zeolite_ polyethylene glycol octadecyl ether | 20.00 | - | 30.00 | 23.00 | Acetone / 12.50 | 40.20 |
| 23 | Hierarchical zeolite_ Pluronic F127 | 20.00 | - | 24.00 | 23.00 | Acetone / 12.50 | 32.60 |
| 24 | Hierarchical zeolite_ Pluronic F127 | 40.00 | - | 24.00 | 23.00 | Acetone / 12.50 | 66.10 |
| 25 | Hierarchical zeolite_ Pluronic F127 | 60.00 | - | 24.00 | 23.00 | Acetone / 12.50 | 89.90 |
| 26 | Hierarchical zeolite_ Pluronic F127 | 20.00 | - | 24.00 | 23.00 | Acetone / 2.50 | 22.00 |
| 27 | Hierarchical zeolite_ Pluronic F127 | 20.00 | - | 24.00 | 23.00 | Ethyl alcohol/ 2.50 | 21.00 |
| 28 | Hierarchical zeolite_ Pluronic F127 | 20.00 | - | 24.00 | 23.00 | Ethyl alcohol/ 12.5 0 | 23.00 |
| 29 | Hierarchical zeolite_ Pluronic F127 | 20.00 | - | 24.00 | 23.00 | n-butanol/ 2.50 | 14.50 |

(continued)

| Example | Material | Amount of turmerc [% w/w] | Amount of piperine [% w/w] | Stirring time [h] | Stirring temperature [°C] | Type/ amount of solvent [% w/w] | Turmeric loading degree [%] |
|---------|----------|---------------------------|-----------------------------|-------------------|----------------------------|--------------------------------|------------------------------|
| 30 | Hierarchical zeolite_ Pluronic F127 | 20.00 | - | 24.00 | 23.00 | n-butanol/ 12.5 0 | 17.50 |
| 31 | Hierarchical zeolite_ Pluronic F127 | 20.00 | - | 24.00 | 20.00 | Acetone/ 12.50 | 30.00 |
| 32 | Hierarchical zeolite_ Pluronic F127 | 20.00 | - | 24.00 | 40.00 | Acetone / 12.50 | 31.40 |
| 33 | Hierarchical zeolite_ Pluronic F127 | 20.00 | - | 30.00 | 23.00 | Acetone / 12.50 | 33.40 |
| 34 | Hierarchical zeolite_ hexadecyltrimethylammonium bromide | 20.00 | 0.10 | 24.00 | 23.00 | Acetone / 12.50 | 39.50 |
| 35 | Hierarchical zeolite_ hexadecyltrimethylammonium bromide | 20.00 | 0.20 | 24.00 | 23.00 | Acetone / 12.50 | 47.50 |
| 36 | Hierarchical zeolite_ hexadecyltrimethylammonium bromide | 20.00 | 0.40 | 24.00 | 23.00 | Acetone / 12.50 | 31.70 |
| 37 | Hierarchical zeolite_ polyethylene glycol octadecyl ether | 20.00 | 0.10 | 24.00 | 23.00 | Acetone / 12.50 | 81.60 |
| 38 | Hierarchical zeolite_ polyethylene glycol octadecyl ether | 20.00 | 0.20 | 24.00 | 23.00 | Acetone / 12.50 | 84.70 |
| 39 | Hierarchical zeolite_ polyethylene glycol octadecyl ether | 20.00 | 0.40 | 24.00 | 23.00 | Acetone / 12.50 | 39.00 |
| 40 | Hierarchical zeolite_ Pluronic F127 | 20.00 | 0.10 | 24.00 | 23.00 | Acetone / 12.50 | 41.30 |
| 41 | Hierarchical zeolite_ Pluronic F127 | 20.00 | 0.20 | 24.00 | 23.00 | Acetone / 12.50 | 70.90 |
| 42 | Hierarchical zeolite_ Pluronic F127 | 20.00 | 0.40 | 24.00 | 23.00 | Acetone / 12.50 | 33.90 |

According to the examples 43-93 a high degree of turmeric release was obtained, as demonstrated in table 2

| Example | Amount of turmeric in relation to zeolite [% w/w] | Hierarchical zeolite with turmeric: amount of phosphate buffer | pH | Penetration promoter/ Contents % in relation to the phosphate buffer | Time [h] | Temperature [°°C] | Radiation wavelength range [nm] | Turmeric release degree [%] |
|---------|---------------------------------------------------|-----------------------------------------------------------------|-----|------------------------------------------------------------------------|----------|-------------------|----------------------------------|------------------------------|
| 43. | | 1:10 | 5.8 | - | 24 | 37 | 400-800 | 3.25 |

(continued)

| Example | Amount of turmeric in relation to zeolite [% w/w] | Hierarchical zeolite with turmeric: amount of phosphate buffer | pH | Penetration promoter/ Contents % in relation to the phosphate buffer | Time [h] | Temperature [°°C] | Radiation wavelength range [nm] | Turmeric release degree [%] |
|---|---|---|---|---|---|---|---|---|
| 44. | | 1:10 | 5.8 | Glycerine/25 | 24 | 37 | 400-800 | 45.60 |
| 45. | | 1:10 | 5.0 | Glycerine/25 | 24 | 37 | 400-800 | 38.00 |
| 46. | | 1:10 | 6.0 | Glycerine/25 | 24 | 37 | 400-800 | 32.00 |
| 47. | | 1:5 | 5.8 | Glycerine/25 | 24 | 37 | 400-800 | 43.00 |
| 48. | | 1:15 | 5.8 | Glycerine/25 | 24 | 37 | 400-800 | 28.00 |
| 49. | | 1:10 | 5.8 | Glycerine/5 | 24 | 37 | 400-800 | 7.50 |
| 50. | | 1:10 | 5.8 | Ethyl alcohol/25 | 24 | 37 | 400-800 | 4.58 |
| 51. | 60 | 1:10 | 5.8 | Ethyl alcohol/5 | 24 | 37 | 400-800 | 3.89 |
| 52. | | 1:10 | 5.8 | Tween 80/25 | 24 | 37 | 400-800 | 14.56 |
| 53. | | 1:10 | 5.8 | Tween 80/5 | 24 | 37 | 400-800 | 9.14 |
| 54. | | 1:10 | 5.8 | Glycerine/25 | 0.5 | 37 | 400-800 | 7.07 |
| 55. | | 1:10 | 5.8 | Glycerine/25 | 30 | 37 | 400-800 | 21.31 |
| 56. | | 1:10 | 5.8 | Glycerine/25 | 24 | 35 | 400-800 | 38.00 |
| 57. | | 1:10 | 5.8 | Glycerine/25 | 24 | 43 | 400-800 | 15.11 |
| 58. | | 1:10 | 5.8 | Glycerine/25 | 24 | 37 | 200-400 | 12.57 |
| 59. | | 1:10 | 5.8 | Glycerine/25 | 24 | 35 | - | 6.99 |
| 60. | | 1:10 | 5.8 | -- | 24 | 37 | 400-800 | 1.88 |
| 61. | | 1:10 | 5.8 | Glycerine/25 | 24 | 37 | 400-800 | 19.75 |
| 62. | | 1:10 | 5.0 | Glycerine/25 | 24 | 37 | 400-800 | 18.65 |
| 63. | 40 | 1:10 | 6.0 | Glycerine/25 | 24 | 37 | 400-800 | 17.98 |
| 64. | | 1:5 | 5.8 | Glycerine/25 | 24 | 37 | 400-800 | 19.50 |
| 65. | | 1:15 | 5.8 | Glycerine/25 | 24 | 37 | 400-800 | 13.87 |
| 66. | | 1:10 | 5.8 | Glycerine/5 | 24 | 37 | 400-800 | 2.25 |

(continued)

| Example | Amount of turmeric in relation to zeolite [% w/w] | Hierarchical zeolite with turmeric: amount of phosphate buffer | pH | Penetration promoter/ Contents % in relation to the phosphate buffer | Time [h] | Temperature [°°C] | Radiation wavelength range [nm] | Turmeric release degree [%] |
|---|---|---|---|---|---|---|---|---|
| 67. | | 1:10 | 5.8 | Ethyl alcohol/ 25 | 24 | 37 | 400-800 | 3.99 |
| 68. | | 1:10 | 5.8 | Ethyl alcohol/ 5 | 24 | 37 | 400-800 | 3.02 |
| 69. | | 1:10 | 5.8 | Tween 80/25 | 24 | 37 | 400-800 | 8.98 |
| 70. | | 1:10 | 5.8 | Tween 80/5 | 24 | 37 | 400-800 | 7.12 |
| 71. | | 1:10 | 5.8 | Glycerine/25 | 0.5 | 37 | 400-800 | 5.02 |
| 72. | | 1:10 | 5.8 | Glycerine/25 | 30 | 37 | 400-800 | 15.67 |
| 73. | | 1:10 | 5.8 | Glycerine/25 | 24 | 35 | 400-800 | 24.58 |
| 74. | | 1:10 | 5.8 | Glycerine/25 | 24 | 43 | 400-800 | 10.68 |
| 75. | | 1:10 | 5.8 | Glycerine/25 | 24 | 37 | 200-400 | 8.97 |
| 76. | | 1:10 | 5.8 | Glycerine/25 | 24 | 35 | - | 4.50 |
| 77. | | 1:10 | 5.8 | -- | 24 | 37 | 400-800 | 1.47 |
| 78. | | 1:10 | 5.8 | Glycerine/25 | 24 | 37 | 400-800 | 10.18 |
| 79. | | 1:10 | 5.0 | Glycerine/25 | 24 | 37 | 400-800 | 9.76 |
| 80. | | 1:10 | 6.0 | Glycerine/25 | 24 | 37 | 400-800 | 9.02 |
| 81. | | 1:5 | 5.8 | Glycerine/25 | 24 | 37 | 400-800 | 10.12 |
| 82. | | 1:15 | 5.8 | Glycerine/25 | 24 | 37 | 400-800 | 7.22 |
| 83. | | 1:10 | 5.8 | Glycerine/5 | 24 | 37 | 400-800 | 1.87 |
| 84. | | 1:10 | 5.8 | Ethyl alcohol/ 25 | 24 | 37 | 400-800 | 3.80 |
| 85. | 20 | 1:10 | 5.8 | Ethyl alcohol/ 5 | 24 | 37 | 400-800 | 2.98 |
| 86. | | 1:10 | 5.8 | Tween 80/25 | 24 | 37 | 400-800 | 6.87 |
| 87. | | 1:10 | 5.8 | Tween 80/5 | 24 | 37 | 400-800 | 5.89 |
| 88. | | 1:10 | 5.8 | Glycerine/25 | 0.5 | 37 | 400-800 | 4.78 |
| 89. | | 1:10 | 5.8 | Glycerine/25 | 30 | 37 | 400-800 | 13.43 |
| 90. | | 1:10 | 5.8 | Glycerine/25 | 24 | 35 | 400-800 | 18.98 |
| 91. | | 1:10 | 5.8 | Glycerine/25 | 24 | 43 | 400-800 | 8.67 |
| 92. | | 1:10 | 5.8 | Glycerine/25 | 24 | 37 | 200-400 | 6.45 |
| 93. | | 1:10 | 5.8 | Glycerine/25 | 24 | 35 | - | 3.90 |

## Claims

1. A method of applying turmeric on hierarchical zeolites, **characterized in that** to hierarchical zeolite possessing secondary porosity, pores in the range 2-50 nm, synthesised on the basis of faujasite and either non-ionic structure forming agents selected from the group of polyethylene glycol octadecyl ether or Pluronic F127 or the ionic structure-forming agent hexadecyltrimethylammonium bromide in the presence or absence of piperine, the following is added:

- a solvent, advantageously acetone in an amount of 2.5-12.5% w/w, advantageously 12.5% w/w in relation to hierarchical zeolite,
- 20-60% w/w of turmeric, advantageously 20% w/w of turmeric in relation to hierarchical zeolite, and
- 0.1-0.4% w/w of piperine, advantageously 0.1% w/w of piperine in relation to hierarchical zeolite,
whereas the entire process of mixing is conducted for 24-30 h advantageously 24 h in a temperature of 20-40°C advantageously in 23°C, and
- washing the obtained product with a water-ethanol solution in a volumetric ration of 1:1 and
- drying afterwards.

2. A method of photodynamic release of turmeric from hierarchical zeolite, consisting of:

- adding phosphate buffer with a pH ranging from 5 to 6, advantageously 5,8 to hierarchical zeolite obtained from faujasite type zeolite with applied turmeric, and **characterised by** secondary porosity, with pores in the range of 2 to 50 nm, obtained on the basis of faujasite type zeolite and ionic structure-forming agents e.g. hexadecyltrimethylammonium bromide,

with turmeric in an amount of 20% to 60% w/w, advantageously 60% w/w in relation to hierarchical zeolite, with the amount of buffer being 1:5, 1:10 and 1:15 by weight compared to hierarchical zeolite with applied turmeric, advantageously 1:10 by weight, and

- adding a penetration promoter in an amount of 5% to 25% w/w in relation to the buffer, where the process of releasing of turmeric from hierarchical zeolite is conducted for 0.5 to 30 h in a temperature of 35 to 43°C, advantageously 37°C with the use of UV-Vis in the wavelength range of 400 to 800 nm in order to obtain the product.

3. A method according to claim 2, **characterized in that** the penetration promoter is glycerine or ethyl alcohol or Tween 80.

**Patentansprüche**

1. Verfahren zum Auftragen von Kurkuma auf hierarchische Zeolithe, **dadurch gekennzeichnet, dass** zu hierarchischem Zeolith von einer sekundären Porosität mit Porengröße im Bereich von 2-50 nm, synthetisiert auf der Basis von Faujasit oder nichtionischen Strukturbildnern, ausgewählt aus der Gruppe von Polyethylenglykoloctadecylether oder Pluronic F127 oder ionischem Strukturbildner Hexadecyltrimethylammoniumbromid in Gegenwart oder Abwesenheit von Piperin, Folgendes hinzugefügt wird:

- ein Lösungsmittel, vorzugsweise Aceton in einer Menge von 2,5-12,5 Gew.-%, vorteilhafterweise 12,5 Gew.-% in Bezug auf hierarchischen Zeolith,
- Kurkuma in einer Menge von 20-60 Gew.-%, vorteilhafterweise 20 Gew.-% in Bezug auf hierarchischen Zeolith, und
- Piperin in einer Menge von 0,1-0,4 Gew.-%, vorteilhafterweise 0,1 Gew.-% im Verhältnis zu hierarchischem Zeolith,
wobei der gesamte Mischvorgang 24-30 Stunden, vorzugsweise 24 Stunden, bei einer Temperatur von 20-40°C, vorzugsweise bei 23°C durchgeführt wird, und
- das erhaltene Produkt mit einer Wasser-Ethanol-Lösung im Volumenverhältnis 1:1 gewaschen und
- anschließend getrocknet wird.

2. Verfahren zur photodynamischen Freisetzung von Kurkuma aus hierarchischem Zeolith,

- bei welchem dem hierarchischen Zeolith Phosphatpuffer von einem pH-Wert im Bereich von 5 bis 6, vorzugsweise 5,8 hinzugefügt wird, wobei das Zeolith vom Faujasit-Typ mit aufgetragenem Kurkuma gewonnen wird,

**gekennzeichnet durch** sekundäre Porosität mit Poren im Größenbereich von 2 bis 50 nm, erhalten auf der Basis von Zeolith vom Faujasit-Typ und ionischen strukturbildenden Mitteln, beispielsweise Hexadecyltrimethylammoniumbromid,
mit Kurkuma in einer Menge von 20 bis 60 Gew.-%, vorteilhafterweise 60 Gew.-%, bezogen auf den hierarchischen Zeolith,
wobei die Gewichtsmenge des Puffers 1:5, 1:10 und 1:15, vorteilhafterweise 1:10 im Vergleich zum hierarchischen Zeolith mit aufgetragenem Kurkuma beträgt, und

- durch Zugabe eines Penetrationspromotors in einer Menge von 5 bis 25 Gew.-%, bezogen auf den Puffer, wobei der Prozess der Freisetzung von Kurkuma aus hierarchischem Zeolith 0,5 bis 30 Stunden lang bei einer Temperatur von 35 bis 43°C, vorteilhafterweise 37°C, unter Verwendung von UV-Vis im Wellenlängenbereich von 400 bis 800 nm, durchgeführt wird, um das Produkt zu erhalten.

3. Verfahren nach Anspruch 2, **dadurch gekennzeichnet, dass** der Penetrationsförderer Glycerin oder Ethylalkohol oder Tween 80 ist.

## Revendications

1. Méthode d'application du curcuma sur des zéolithes hiérarchiques, **caractérisée en ce que** l'on ajoute à une zéolite hiérarchique possédant une porosité secondaire, des pores de l'ordre de 2 à 50 nm, synthétisée à partir de faujasite et d'agents structurants non ioniques choisis dans le groupe de l'éther de polyéthylène glycol stéarylique ou du Pluronic® F-127 ou de l'agent structurant ionique tels que le bromure de cétrimonium (CTAB) en présence ou en l'absence de pipérine, des substances suivantes :

- un solvant, avantageusement de l'acétone, dans une proportion de 2,5 à 12,5% en poids, avantageusement 12,5% en poids par rapport à la zéolithe hiérarchique,
- 20-60% en poids de curcuma, avantageusement 20% en poids de curcuma par rapport à la zéolithe hiérarchique, et
- 0,1-0,4% en poids de pipérine, avantageusement 0,1% en poids de pipérine par rapport à la zéolithe hiérarchique,
alors que l'ensemble du processus de mélange est réalisé pendant 24 à 30 heures, avantageusement 24 heures, à une température de 20 à 40°C, avantageusement 23°C, et
- le lavage du produit obtenu s'effectue avec une solution d'eau et d'éthanol dans un rapport volumétrique de 1:1 et par la suite,
- le séchage.

2. Méthode de libération photodynamique du curcuma à partir d'une zéolithe hiérarchique, consistant en :

- l'ajout d'un tampon phosphate dont le pH est compris entre 5 et 6, avantageusement 5,8, à la zéolithe hiérarchique obtenue à partir d'une zéolithe de type faujasite à laquelle on avait appliqué du curcuma, et

**caractérisée par** une porosité secondaire, avec des pores de 2 à 50 nm, obtenus à partir de zéolithe de type faujasite et d'agents structurants ioniques tels que le bromure de cétrimonium (CTAB), avec du curcuma dans une proportion de 20% à 60% en poids, avantageusement 60% en poids par rapport à la zéolithe hiérarchique,
avec la quantité de tampon étant de 1:5, 1:10 et 1:15 en poids par rapport à la zéolithe hiérarchique avec application de curcuma, avantageusement 1:10 en poids, et

- l'ajout d'un promoteur de pénétration dans une quantité de 5% à 25% en poids, par rapport au tampon, où le processus de libération du curcuma de la zéolithe hiérarchique se réalise pendant 0,5 à 30 h à une température de 35 à 43°C, avantageusement 37°C avec l'utilisation d'UV-Vis dans la gamme de longueurs d'onde de 400 à 800 nm afin d'obtenir le produit.

3. Procédé selon la revendication 2, **caractérisé en ce que** le promoteur de pénétration est la glycérine ou l'alcool éthylique ou le Tween 80.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2020155480 A1
- WO 2001012221 A1

- DE 102011114455 A1

### Non-patent literature cited in the description

- **A. FELICZAK-GUZIK.** Hierarchical zeolites: Synthesis and catalytic properties. *Micropor. Mesopor. Mat.,* 2018, vol. 259, 33-45
- **H. QU ; Y. MA ; B. LI ; L. WANG.** Hierarchical zeolites: synthesis, structural control, and catalytic applications. *Emerg. Mat.,* 2020, vol. 3, 225-245
- **A. G. ABD-ELSATAR ; M. M. FARAG ; H. F. YOUSSEF ; S. A. SALIH ; M. M. MOUNIER ; E. EL-MELIEGY.** Different zeolite systems for colon cancer therapy: monitoring of ion release, cytotoxicity and drug release behavior. *Progress in Biomat,* 2019, vol. 8, 101-113
- **M. J. RATHBONE ; J. HADGRAFT ; M. S. ROBERTS ; M. E. LANE.** Modified-release drug delivery technology. Informa, 2008, vol. 1
- **M. SZNITOWSKA ; W. ZEBROWSKA.** Doustne postacie leku o modyfikowanym uwalnianiu. Cz. I - Tabletki o przedfuionym uwalnianiu - (Oral forms of modified release medication, part I - prolonged release tablets). *Ordynator Leków.,* 2003, vol. 3 (5), 3-14
- **P. HORCAJADA ; A. RÁMILA ; J. PÉREZ-PARIENTE ; M. VALLET-REGI.** *Micropor. Mesopor. Mat.,* 2004, vol. 68, 105-109
- **A. G. ABD-ELSATAR ; M. M. FARAG ; H. F. YOUSSEF ; S. A. SALIH ; M. M. MOUNIER ; E. EL-MELIEGY.** Different zeolite systems for colon cancer therapy: monitoring of ion release, cytotoxicity and drug release behavior. *Progress in,* 2019, vol. 8, 101-113
- **M. KRALJ ; K. PAVELIC.** Medicine on a small scale: how molecular medicine can benefit from self-assembled and nanostructured materials. *EMBO Rep,* 2003, vol. 4, 1008-1012

- **D. C. THOM ; J. E. DAVIES ; J. P. SANTERRE ; S. FRIEDMAN.** The haemolytic and cytotoxic properties of a zeolite-containing root filling material in vitro. *Oral Surg Oral Med Oral Pathol Oral Radiol Endod,* 2003, vol. 95, 101-108
- **E. KHODAVERDI ; H. A. SOLEIMANI ; F. MOHAMMADPOUR ; F. HADIZADEH.** Synthetic zeolites as controlled-release delivery systems for anti-inflammatory drugs. *Chem Biol Drug Des.,* 2016, vol. 87, 849-857
- **A. GOEL ; A. B. KUNNUMAKKARA ; B. B. AGGARWAL.** *Biochem Pharmacol.,* 2008, vol. 75, 787-809
- **K. NAGAHAMA ; T. UTSUMI ; T. KUMANO ; S. MAEKAWA ; N. OYAMA ; J. KAWAKAMI.** *Sci Rep.,* 2016, vol. 60, 30962
- **Y. S. KIM ; Y. AHN ; S. H. HONG ; S. Y. JOO ; K. H. KIM ; I. S. SOHN ; H. W. PARK ; Y. J. HONG ; J. H. KIM ; W. KIM.** *Jour. Cardiovasc. Pharmacol.,* 2007, vol. 50, 41
- **X. L. JUN ; H. XIANG-FE ; L. Z. HANG.** *Anti-Cancer Agents in Medical Chem.,* 2012, vol. 12, 210
- **M. ZHENG ; S. LIU ; X. GUAN ; Z. XIE.** *ACS Applied Materials & Interfaces,* 2015, vol. 7 (40
- **SHOBA G. ; JOY D. ; JOSEPH T. ; MAJEED M. ; RAJENDRAN R. ; SRINIVAS PS.** *Planta Medica,* 1998, vol. 64, 353-356
- **A. FELICZAK-GUZIK ; M. SPRYNSKYY ; I. NOWAK ; M. JARONIEC ; B. BUSZEWSKI.** *Journal of Colloid and Interface Science,* 2018, vol. 516, 379-383
- **A. FELICZAK-GUZIK ; M. SPRYNSKYY ; I. NOWAK ; B. BUSZEWSKI.** *Catalysts,* 2018, vol. 8, 31-43